# EUROPEAN PATENT APPLICATION

(11) **EP 3 510 977 A1**
(43) Date of publication of application: **17.07.2019**
(21) Application number: 18210995.9
(22) Date of filing: 07.12.2018
(51) Int. Cl.: A61F 2/95

(54) **AN ENDOLUMINAL DELIVERY DEVICE ASSEMBLY**

(30) Priority: 10.01.2018 AU 2018900064; 10.01.2018 US 201862615720 P
(71) Applicant: Cook Medical Technologies LLC, Bloomington, IN 47404 (US)
(72) Inventor: COLLINS, James, Paddington, Queensland 4064 (AU); SMITH, Logan, Mount Gravatt, Queensland 4122 (AU)
(74) Representative: Williams Powell

(57) **Abstract**

An endoluminal delivery device assembly (10) comprises: an introducer (500) including a seal housing assembly (540), housing a seal (550), and a sheath (510) extending proximally therefrom; a delivery device (11) including a handle body (218) and a pusher catheter extending from the body, the pusher catheter (300) having a pusher catheter external surface, the pusher catheter (300) extending through the introducer (500) such that the external surface slidably engages with the seal (550); a guide wire catheter (40) extending through the body and through the pusher, the guide wire catheter (40) being affixed at a proximal end thereof to a tip assembly; and a linking portion (710) for linking the body and the seal housing assembly together, the linking portion (710) having an unlinked condition and a linked condition. In the linked condition, sliding movement between the pusher catheter and the seal is limited such that disengagement between the pusher catheter external surface and the seal is prevented.

## Description

### Technical Field

The present invention relates to an endoluminal introducer assembly, for example for delivering and deploying endografts. The preferred embodiment relates to an endoluminal delivery device assembly capable of delivering prostheses, endografts or stent grafts into the vascular system of humans or animals.

### Background of the Invention

Stent graft and delivery devices are used in aortic intervention. They are used by vascular surgeons to treat aneurysms and to repair regions of the aorta, including the aortic arch, the thoracic aorta, the abdominal aorta and the aortic bifurcation.

Delivery devices allow deployment of intraluminal prostheses or endografts into the lumen of a patient from a remote location.

Numerous devices and procedures have been developed that involve the percutaneous insertion of a prosthesis into a body lumen, such as a blood vessel or duct, of a patient's body. Such a prosthesis may be introduced into the lumen by a variety of known techniques. For example, a wire guide may be introduced into a blood vessel using the Seldinger technique. This technique involves creating a surgical opening in the vessel with a needle and inserting a wire guide into the vessel through a bore of the needle. The needle can be withdrawn, leaving the wire guide in place. A delivery device is then inserted over the wire guide and into the vessel. The delivery device may be used in conventional fashion to insert into the blood vessel a variety of prostheses, such as stents, stent grafts, catheters, cardiac leads, balloons, and the like.

For example, the delivery device may be used to deliver and deploy an expandable prosthesis, such as a stent graft, to an aneurysmal blood vessel site. A stent graft is usually formed from a tubular body of a biocompatible graft material with one or more stents mounted into or onto the tubular body to provide support therefor. The stents may be balloon expandable stents and/or self-expanding stents. The deployment of the prosthesis into the lumen of a patient from a remote location by the use of an introducer delivery and deployment device is described in, for example, US-7,435,253 to Hartley entitled "A Prosthesis and a Method and Means of Deploying a Prosthesis," which is incorporated herein by reference in its entirety.

Delivery devices are configured to retain a prosthesis in a delivery configuration during delivery to the desired deployment site. The delivery catheter typically includes an inner catheter/cannula spaced from an outer sheath to define a prosthesis retaining region for receiving the prosthesis. The prosthesis is loaded onto an inner cannula along a prosthesis retaining region, with an outer sheath retaining the prosthesis in the delivery configuration. After the delivery device is delivered to the desired deployment site, the prosthesis may be deployed, for example, with retraction of the outer sheath relative to the inner cannula away from the prosthesis to allow for expansion thereof. Accurate placement of an appropriately sized prosthesis generally sufficiently covers the target site for treatment and the ends of the prosthesis are typically engaged with healthy tissue of the body lumen.

Endovascular delivery devices require significant expertise and experience to operate. Correct sequencing of various manual operations performed outside the body (at a distal end of a delivery device) are required for successful and optimum deployment of an endograft. It is desirable to make operation as intuitive and foolproof as possible.

For endovascular delivery devices that include a pusher catheter and a pusher catheter seal, uncontrolled or inadvertent withdrawal of the pusher catheter through the pusher catheter seal can lead to sealing problems and increase the likelihood or severity of blood loss where the devices are used within arteries.

Throughout this specification, the term "distal" with respect to a portion of the aorta, a deployment device or an endograft means the end of the aorta, deployment device or endograft further away in the direction of blood flow from the heart and the term "proximal" means the portion of the aorta deployment device or end of the endograft nearer to the heart in the direction of blood flow.

### Summary of the Invention

The present invention seeks to provide an improved endoluminal introducer assembly and improved deployment of endoluminal medical devices.

According to an aspect of the invention, an endoluminal delivery device assembly comprises:
an introducer, the introducer including a seal housing assembly and an elongate sheath extending proximally from the seal housing assembly, the seal housing assembly housing a pusher catheter seal;
a delivery device, the delivery device including a handle body and an elongate pusher catheter extending proximally from the handle body, the pusher catheter having a pusher catheter external surface, the pusher catheter extending through the introducer such that the pusher catheter external surface slidably engages with the pusher catheter seal;
a tip assembly at a proximal end of the delivery device;
a guide wire catheter extending through the handle body and through the pusher, the guide wire catheter being affixed at a proximal end thereof to the tip assembly;
an endograft receiving portion for receiving the endograft between the tip assembly and the pusher; and
a linking portion for linking the handle body and the seal housing assembly together, the linking portion mounted between the handle body and the seal housing assembly, the linking portion having an unlinked condition and a linked condition,
whereby, in the linked condition, sliding movement between the pusher catheter and the pusher catheter seal is limited such that disengagement between the pusher catheter external surface and the pusher catheter seal is prevented.

The linking portion may be slidably mounted to the handle body.

Advantageously, when in the linked condition, the linking portion is joined to the seal housing assembly.

The assembly may comprise a joiner assembly for joining the linking portion to the seal housing assembly.

The joiner assembly may comprise:
a first mating joiner feature on the linking portion; and
a second mating joiner feature on seal housing assembly.

Advantageously, the pusher catheter includes a proximal end, and wherein the assembly comprises:
an first condition in which the proximal end of the pusher catheter is spaced from the tip assembly at a first spacing for accommodating a compressed endograft; and
a second condition in which the proximal end of the pusher catheter is spaced from the tip assembly at a second spacing, the second spacing shorter than the first spacing.

The sheath may include a proximal end, and wherein the assembly has a third condition in which the proximal end of the sheath is adjacent to the tip assembly.

Advantageously, when in the linked condition, the limited sliding movement between the pusher catheter and the pusher catheter seal is sufficient to bring the proximal end of the sheath into engagement with the tip assembly.

The assembly may further comprise a lock, the lock comprising :
a locking actuator having a locking projection; and
a locking recess,
wherein the lock has a locked condition in which the locking projection engages the locking recess thereby locking the linking portion to the handle body so as to prevent relative sliding movement.

The actuator is advantageously moveable to an unlocked condition in which the locking projection is disengaged from the locking recess.

In other embodiments, when in the unlinked condition, the linking portion is slidable mounted to the seal housing assembly.

Advantageously, the linking portion is slidably mounted to the seal housing assembly.

When in the linked condition, the linking portion may be joined to the handle body.

The assembly may further comprise a joiner assembly for joining the linking portion to the handle body.

The joiner assembly may comprise:
a first mating joiner feature on the linking portion; and
a second mating joiner feature on handle body.

### Brief Description of the Drawings

Embodiments of the present invention are described below, by way of example only, with reference to the accompanying drawings, in which:
Figure 1A shows in side view an embodiment of an endoluminal delivery device assembly according to the invention;
Figure 1B shows a proximal end of the assembly of Figure 1A;
Figure 2A is a similar side view to that of Figure 1A showing a sheath assembly in a retracted position;
Figure 2B shows a proximal end of the assembly of Figure 2A;
Figure 3A is a similar side view to that of Figure 2A showing an endograft in a partially released condition;
Figure 3B shows a proximal end of the assembly of Figure 3A;
Figure 4A is a similar side view to that of Figure 3A but showing an endograft in a fully released condition;
Figure 4B shows a proximal end of the assembly of Figure 4A;
Figure 5A is a similar side view to that of Figure 4A but showing a tip assembly of the delivery assembly retracted back into engagement with a proximal end of a sheath;
Figure 5B shows a proximal end of the assembly of Figure 5A;
Figure 6 is a close-up isometric view showing the delivery assembly of Figure 1A;
Figure 7 is a similar view to that of Figure 6, showing the delivery assembly after its sheath assembly has been withdrawn into the position shown in Figure 2A;
Figure 8 is a close-up isometric view similar to that of Figure 7;
Figure 9 is a detailed cross-sectional view showing the delivery assembly just before engagement of the sheath assembly with the handle assembly;
Figure 10A is a similar view to that of Figure 9, showing the delivery assembly after engagement of the sheath assembly with the handle assembly;
Figure 10B is a close up view of the circled portion 10B of Figure 10A;
Figure 11 is a close-up isometric view of the assembly shown in Figure 5A in the configuration shown in Figure 5A;
Figure 12 is a similar view to Figure 11, showing the detailed side view of a portion of the assembly illustrated in Figure 11;
Figure 13 shows a portion of the assembly in the position shown in Figure 12, in a detailed cross-sectional view;
Figures 14A and 14B show close-up isometric views of a proximal end of a handle body that can be seen in Figures 11 and 12;
Figures 15A and 15B are end and isometric views respectively of a linking portion of the delivery assembly shown in Figures 11 and 12 for instance;
Figure 16 is a detailed isometric view of a component of the handle body shown in Figures 14A and 14B;
Figures 17A and 17B show components of the proximal end of the handle body shown in Figures 14A and 14B;
Figure 18 is a detailed isometric view showing the inner handle portion of the assembly shown in Figures 1A to 5A and Figure 9;
Figure 19 is an isometric view of another embodiment of a delivery assembly according to the invention in a position corresponding to the position of the first embodiment shown in Figure 1A;
Figure 20 is a similar view to that of Figure 19, showing the delivery assembly after its sheath assembly has been withdrawn into the position corresponding to the position of the first embodiment shown in Figure 2A;
Figure 21 is a detailed cross-sectional view showing the delivery assembly just before mating of joiner features;
Figures 22A is a similar detailed cross-sectional view to Figure 21, showing the delivery assembly just after mating of joiner features;
Figures 22B is a close up view of a portion of Figure 22A;
Figure 23 shows the assembly in a position corresponding to the position of the first embodiment shown in Figure 4A in the configuration shown in Figure 4A; and
Figure 24 shows the assembly in a position corresponding to the position of the first embodiment shown in Figure 5A in the configuration shown in Figure 5A.

### Detailed Description

For the purposes of understanding the principles of the teachings herein, reference will now be made to the embodiments illustrated in the drawings, and specific language will be used to describe them. It is to be understood that the Figures are, in some cases, schematic and do not show the various components in their actual scale. In many instances, the Figures show scaled up components to assist the reader.

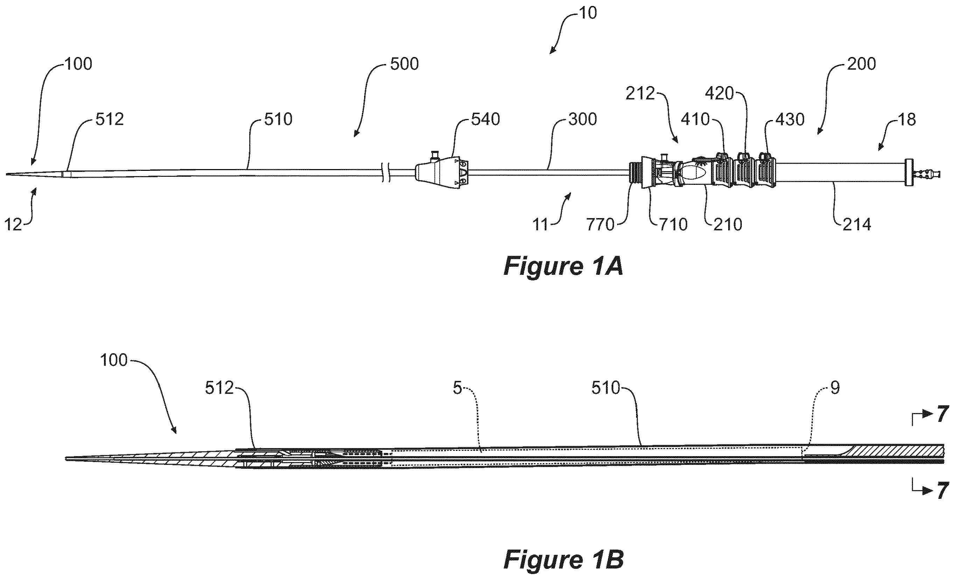

Referring now to Figures 1A, 1B, 2A, 2B, 3A, 3B, 4A, 4B, 5A, 5B, 6 and 7, there is shown an endovascular delivery device assembly 10 for the percutaneous insertion into an artery (or other bodily lumen) of medical devices or prostheses such as stents, stent grafts, catheters, cardiac leads, balloons, and the like, according to an embodiment of the invention.

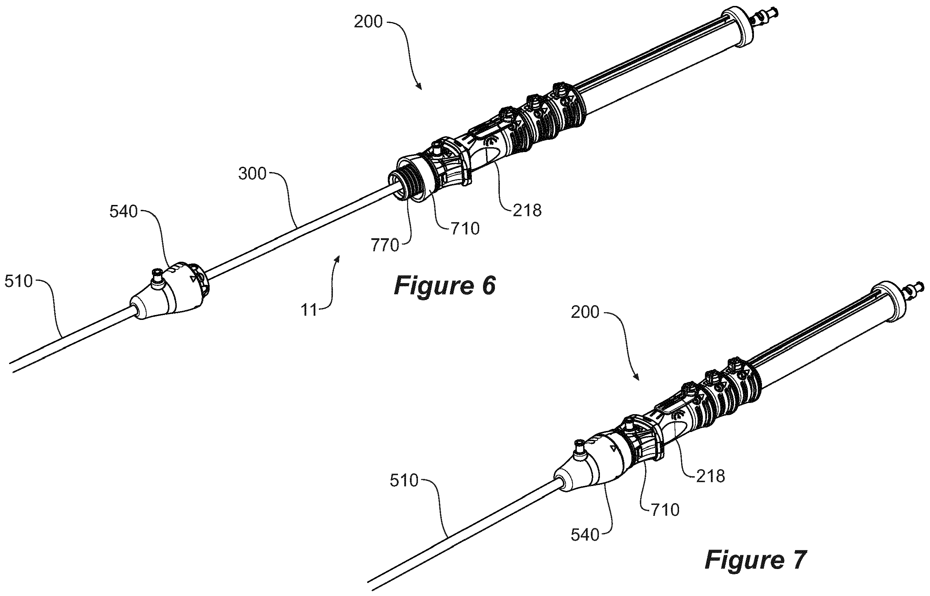

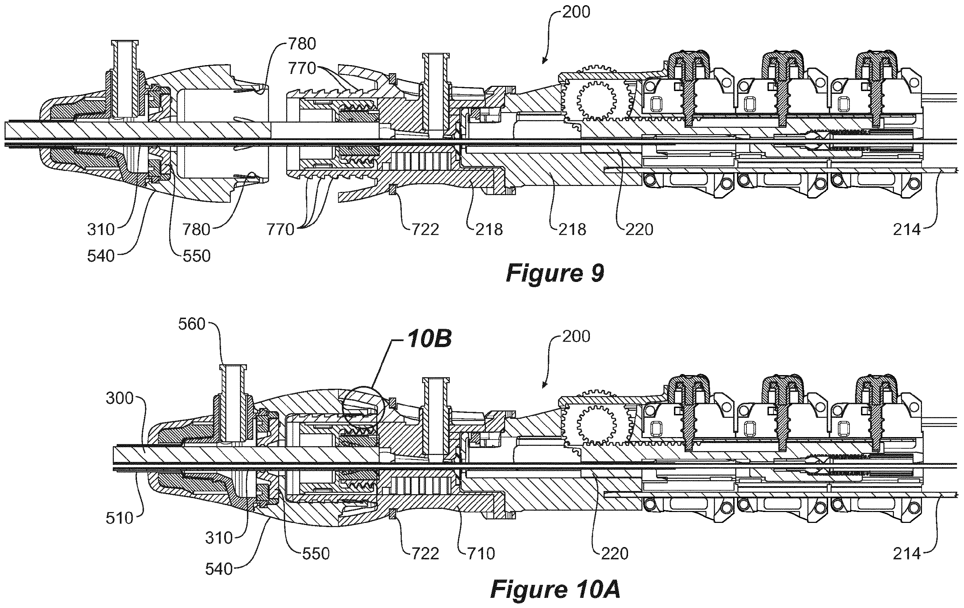

Referring again to Figure 1A, in conjunction with Figures 6 and 7, it can be seen that the endovascular delivery device assembly 10 comprises: a sheath assembly or introducer 500 and a delivery device 11. The introducer 500 includes a seal housing assembly 540 and an elongate sheath 510 extending proximally from the seal housing assembly. The seal housing assembly houses a pusher catheter seal 550 as shown in the cross-sectional views of Figures 9 and 10A.

It can be seen from Figure 1B that the delivery device 11 is loaded with an endograft or stent graft 5.

Referring to Figures 1A and 1B, it can be seen that the device has a proximal end 12 and a distal end 18. The delivery device 11 has an outer handle portion 210 mounted to its distal end 18. The outer handle portion 210 has a handle body 218 and a track 214 extending distally from the handle body 218. The handle body 218 is more clearly shown in the cross-sectional view of Figure 9.

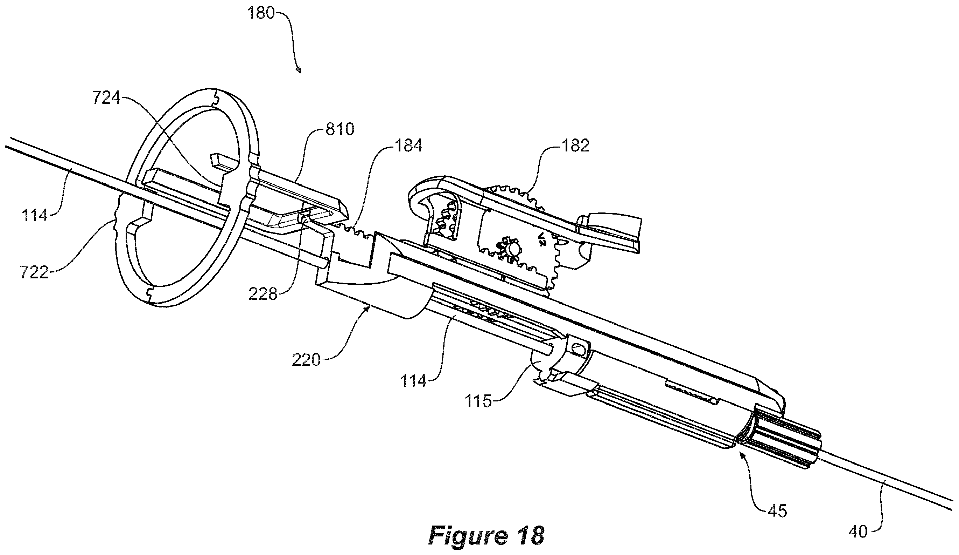

The delivery device 11 also has an inner handle portion 220 which is shown in Figures 9 and Figure 18. The inner handle portion 220 is slidably moveable within, and with respect to, the outer handle portion 210 from a distal position to a proximal position.

A tip assembly 100 is located at the proximal end 12 of the delivery device 11, as is shown in Figures 1A and 1B.

The delivery device 11 also has an elongate pusher catheter 300 extending proximally from the handle body 218 towards the tip assembly 100, as can be seen in Figures 1B, 2B, 3B and 4B. A distal end of the pusher catheter is attached to the handle body 218. The pusher catheter has a pusher catheter external surface 310, and the pusher catheter extends through the introducer such that the pusher catheter external surface slidably engages with the pusher catheter seal 550 as can be seen in Figure 9.

A guide wire catheter 40 extends through the inner handle portion 220 and through the pusher catheter 300. The guide wire catheter 40 is affixed at a distal end thereof to the inner handle portion 220 and is affixed at a proximal end 12 thereof to the tip assembly 100. A pin vice assembly 45, the position of which is shown in Figure 18, can be used. Such a pin vice assembly attaches to the inner handle portion 220, as is shown in Figure 18. Various pin vice constructions may be used, such as is described in US-7,435,253 to Hartley referred to above, where the pin vice has a screw cap which, when screwed in, clamps vice jaws against the thin walled metal tube (guide wire catheter).

In other embodiments, not shown, gluing, crimping or other attachment means may be used to secure the guide wire catheter 40 to the inner handle portion 220.

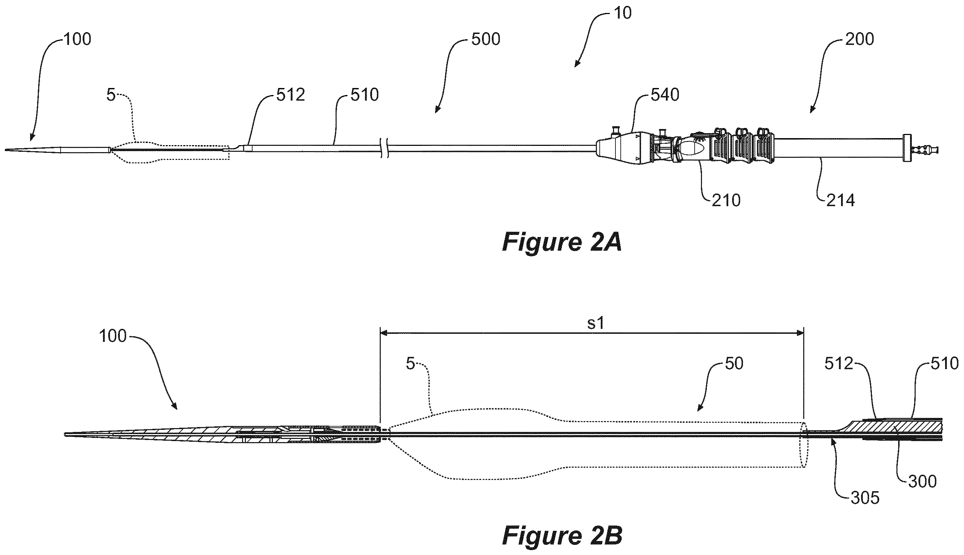

An endograft receiving portion 50, for receiving the endograft 5 between the tip assembly and the pusher catheter, can be seen in Figure 2B. It should be understood that 'between' in this context, means that at least a portion of the endograft receiving portion 50 is between the tip assembly 100 and the pusher catheter 300.

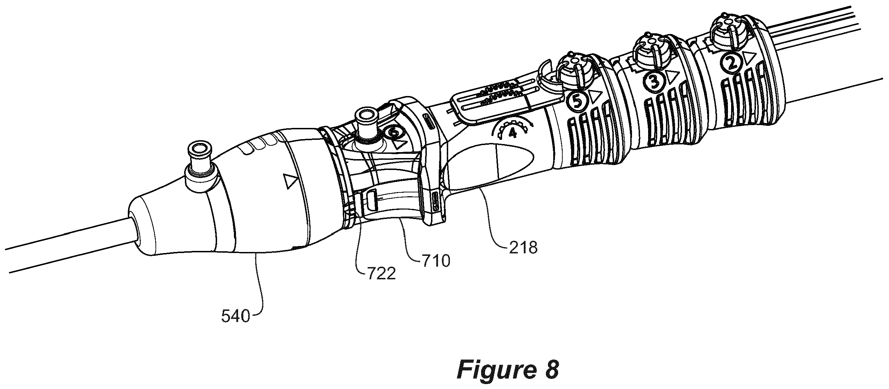

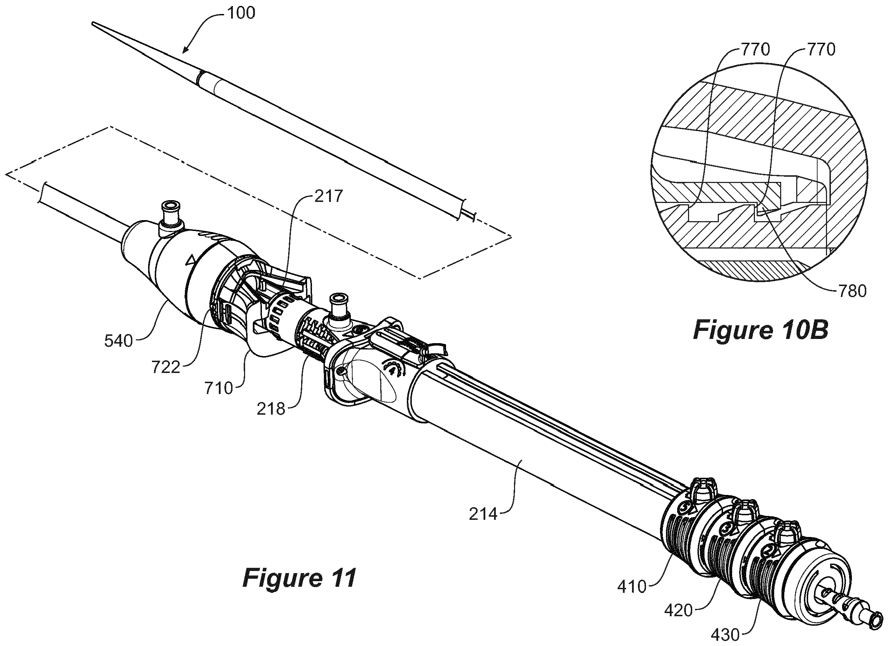

Referring to Figures 6, 7 and 8, it can be seen that the assembly further comprises a linking portion 710 for linking the handle body and the seal housing assembly 540 together. The linking portion 710 is mounted between the handle body 218 and the seal housing assembly 540. Referring now to Figures 9 and 10, it can be seen that the linking portion 710 has an unlinked condition, shown in Figures 6 and 9, and a linked condition, shown in Figures 7, 8, 10A and 10B. In the linked condition, sliding movement between the pusher catheter 300 and the pusher catheter seal 550 is limited such that disengagement between the pusher catheter external surface 310 and the pusher catheter seal 550 is prevented.

Figures 10A and 10B shows that in the linked condition, the linking portion 710 is joined to the seal housing assembly 540.

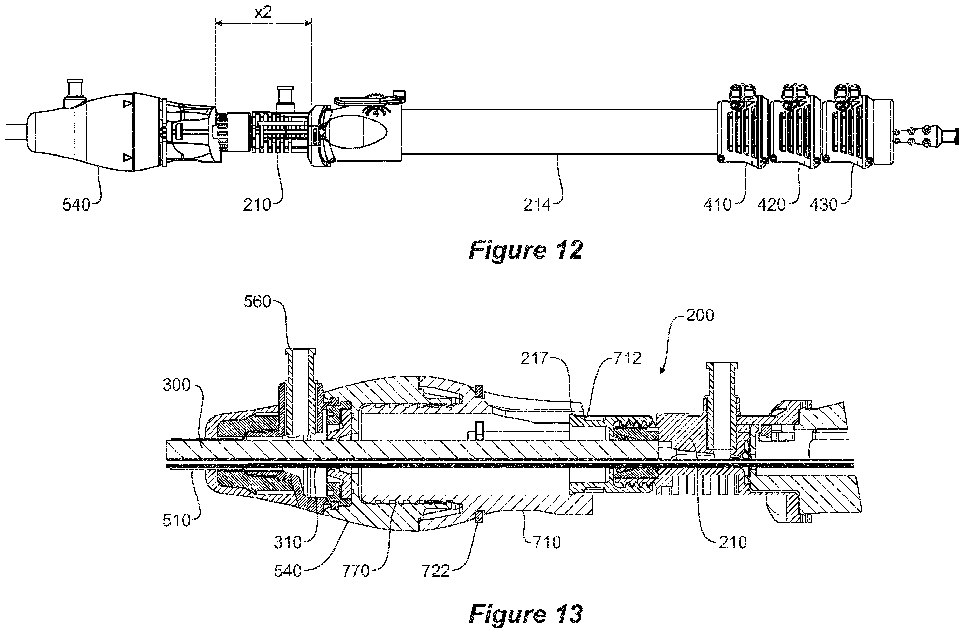

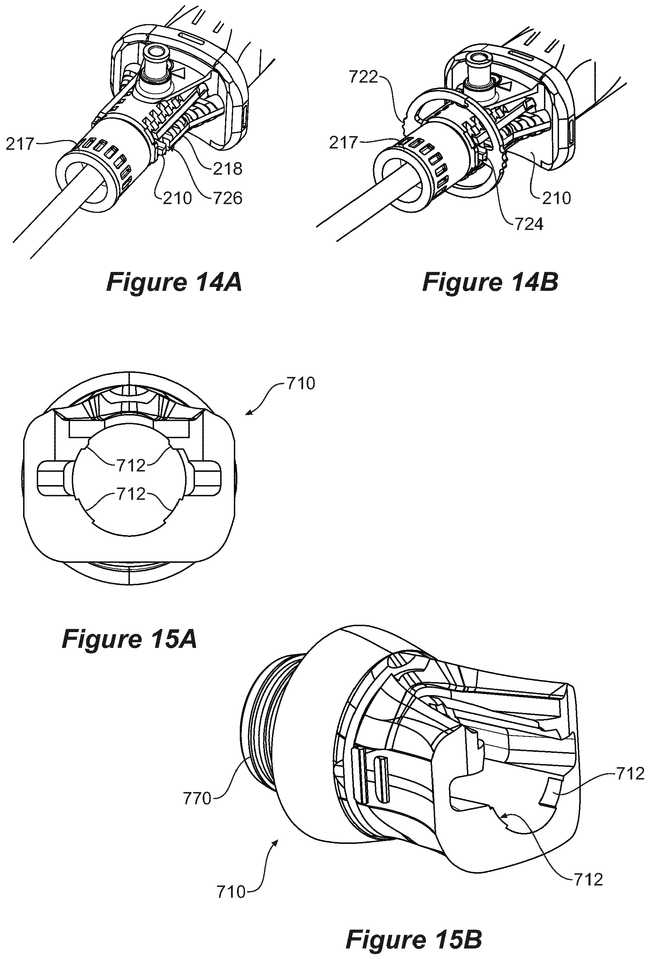

The linking portion 710 is shown as an isolated component in Figures 15A and 15B. In these Figures, it can be seen that the linking portion 710 includes four extension limiting projections in the form of tabs 712. These tabs 712 are arranged to engage with a corresponding flange 217 located at a proximal end of the handle body 218 of the outer handle portion 210. The flange 217 is most clearly shown in Figures 14A and 14B. Figures 11, 12 and 13, particularly Figure 13, shows how the flange 217 and the tabs 712 engage to limit the sliding movement between the pusher catheter 300 and the pusher catheter seal 550, once the linking portion 710 has been joined to the seal housing assembly 540 as is described below and is shown, for instance, in Figures 10A and 10B.

The assembly 10 comprises a joiner assembly for joining the linking portion 710 to the seal housing assembly 540. In the embodiment illustrated in the Figures described above, including Figures 6 to 10, the joiner assembly comprises a first mating joiner feature 770 on the linking portion 710. The first mating joining feature 770 is shown in Figure 1A, Figure 6, the cross-sectional view of Figure 13 and in the isometric view Figure of 15B. The joiner assembly further comprises a second mating joining feature 780, which is most easily seen in Figure 9.

Now referring to Figures 9 and 10A, it can be seen that in Figure 9 the mating feature 780 is longitudinally spaced apart from the first mating feature 770. In Figures 10A and 10B, the mating features 780 and 770 are in engagement.

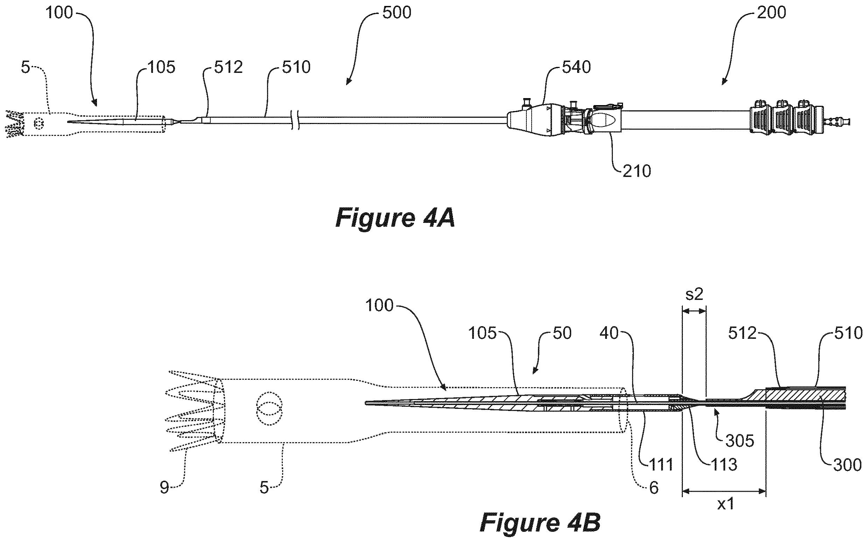

Referring again to Figures 2A and 2B, it can be seen that the pusher catheter 300 has a proximal end 305. It can also be seen that the assembly 10 has a first condition, as shown in Figures 2A and 2B, in which the proximal end 305 of the pusher catheter 300 is spaced from the tip assembly 100 at a first spacing s1 for accommodating a compressed endograft 5. The assembly further has a second condition in which the proximal end 305 of the pusher catheter 300 is spaced from the tip assembly 100 at a second spacing s2, as is illustrated in Figure 4B. The second spacing s2 is shorter than the first spacing s1. Furthermore, the second spacing s2 may be substantially zero. That is, the second spacing s2 may be an engagement, depending on the configuration of the tip assembly 100.

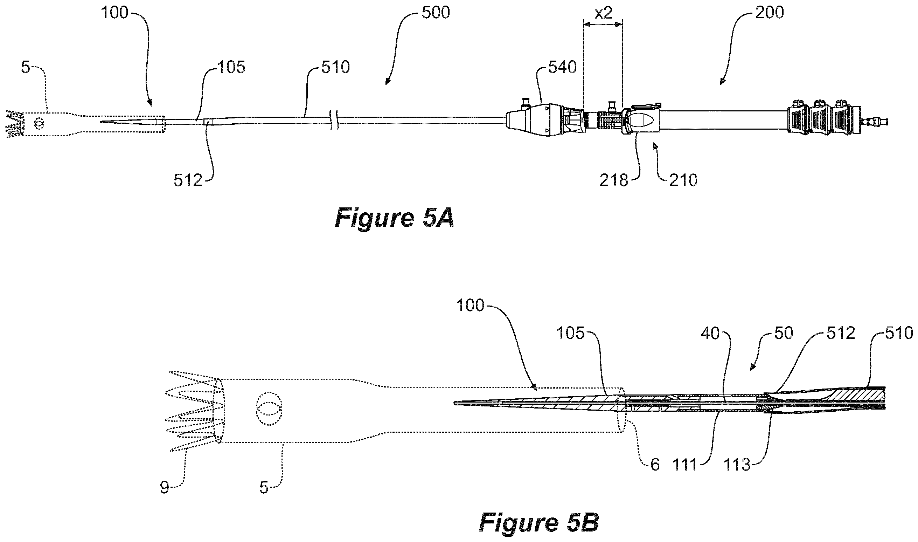

The sheath 510 includes a proximal end 512 shown in Figure 4B. The assembly 10 has a third condition in which the proximal end 512 of the sheath 510 is adjacent to the tip assembly 100, as is shown in Figure 5B.

In the linked condition, the limited sliding movement between the pusher catheter 300 and the pusher catheter seal 550 is sufficient to bring the proximal end 512 of the sheath 510 into engagement with the tip assembly 100, as is shown in Figures 5A and 5B. This distance is indicated by the length x1 shown in Figures 5A and 12.

Referring again to Figures 9, 10A, 12 and 13, a lock comprising a locking actuator 722 is shown. The locking actuator 722 has a locking projection 724 which engages with a locking recess 726. The locking recess is shown in Figure 14A. In Figure 14B, the locking projection 724 can be seen in its engaged position within the locking recess 726. Reading Figures 9, 10, 14A and 14B together, it can be seen that the lock comprises the locking actuator 722 and the locking recess 726. In the locked condition, shown in Figures 9, 10A and 14B, the locking projection 724 engages the locking recess 726 to the handle body 218 so as to prevent relative sliding movement. That is, to prevent relative sliding movement between the linking portion 710 and the handle body 218.

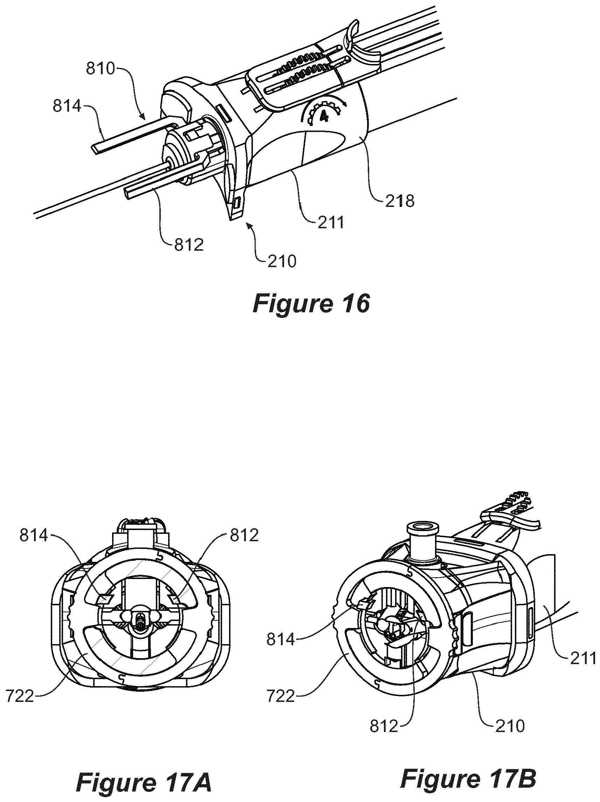

Figures 17A and 17B also show the locking actuator 722 and its mounting position.

The actuator 722 is movable to an unlocked condition in which the locking projection 724 is disengaged from the locking recess 726. This is achieved through rotation of the locking actuator 722. In the unlocked condition, shown in Figures 12 and 13, the linking portion 710 and the handle body 218 are unlocked so as to allow relative sliding movement to the position shown in Figures 12 and 13 from the position shown in Figures 10A and 10B.

It is important to note that in a preferred embodiments of the invention, illustrated and described herein, the length of the above-mentioned sliding movement is arranged to match the distance required to move from the position shown in Figure 4B to the position shown in Figure 5B. That is, the distance x2 shown in Figure 12 is the same, or about the same, as x1 shown in Figure 4B. Preferably, x2 is just a little longer than x1 so that the sheath 510 and the tip assembly 100 marry up, as is shown in Figure 5B with slight overlap. The configuration shown in Figure 5B allows the whole assembly 10 to be withdrawn from the patient with an atraumatic profile, thereby reducing risks of engagement or catching of either deployed endograft(s), plaque or surfaces within the anatomy of the patient.

Referring to Figure 16, which is a detailed isometric view of a component of the handle body shown in Figures 14A and 14B, it can be seen that the handle body 218 has a grip portion 211 (also shown in Figure 17B).

Figure 16 also shows an optional interlock body 810. The interlock body 810 has legs 812 and 814 that extend longitudinally to a position adjacent to the locking projections 724 of the locking actuator 722 as can be seen in Figures 17A, 17B and 18. The interlock body 810 prevents premature actuation of the locking actuator 722. The locking actuator 722 can only be rotated (actuated) after the interlock body 810 has been pulled in a distal direction by the hook 228 shown in Figure 18. This pulling action only occurs when tip has been retrieved through actuation of the tip retriever. In the embodiment shown in Figure 18, the tip is retrieved by rotation of actuator 182 (other actuation devices including those employing levers, racks and pinions may also be used).

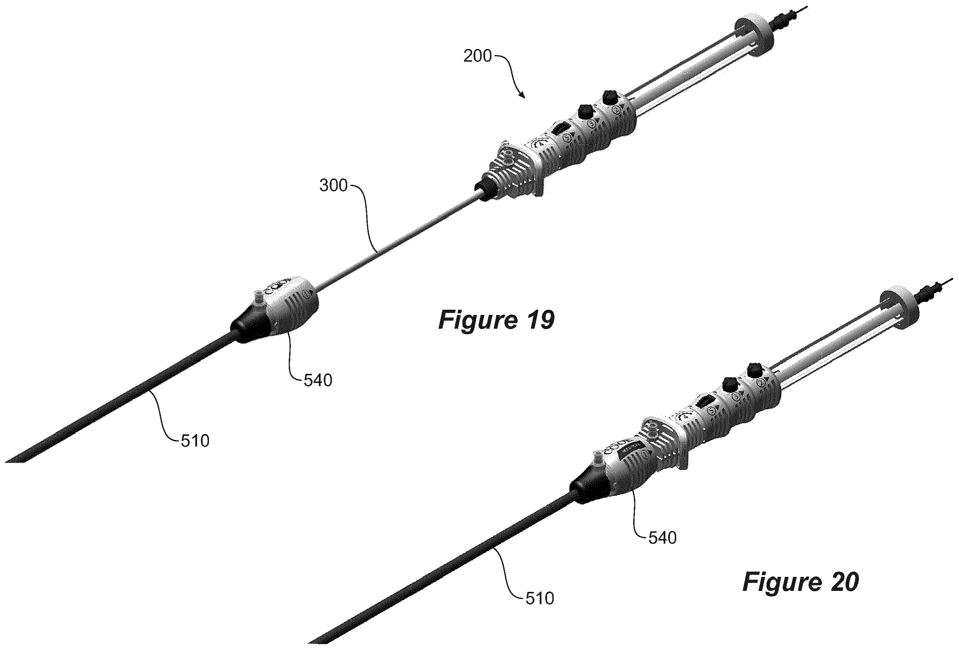

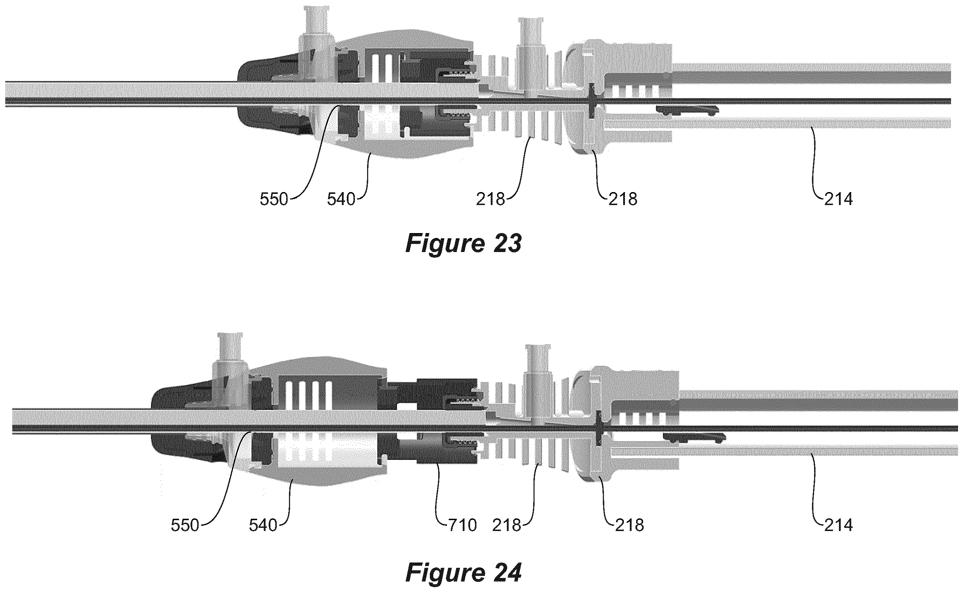

Another embodiment is now be described with reference to Figures 19 to 24.

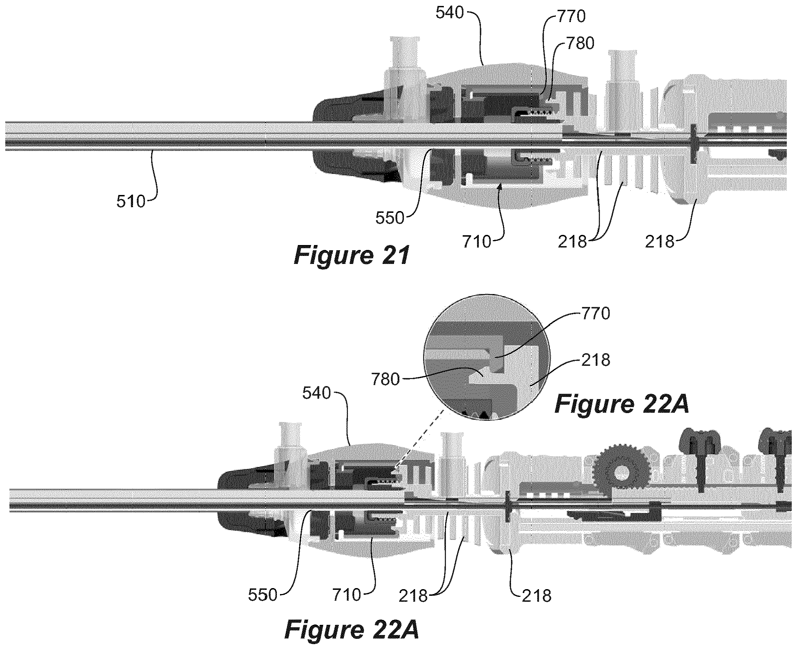

This embodiment is of a generally similar construction to the embodiment of Figures 1 to 18 but differs particularly in the area of the linking portion. With the first-described embodiment, the linking portion 710 is shown as a separate component in Figures 15A and 15B and is generally external in that it sits around or external to the handle body 218. In contrast, the linking portion 710 of the embodiment of Figures 19 to 24, which is most clearly shown in the magnified view of Figure 21, is internal in the sense that it sits more inside the valve body 540. Functionally, the linking portion 710 is similar.

Referring to Figure 21, it can be seen that the linking portion 710 of this embodiment is slidably mounted to the seal housing assembly 540. In Figure 21, the linking portion is not yet in its linked condition. In transitioning from the position illustrated in Figure 21 to the position shown in Figures 22A and 22B, linking occurs. That is, the linking portion 710 links to the handle body 218. More specifically, linking occurs through the use of a joiner assembly adjoining the linking portion to the handle body. The joiner assembly comprises the first mating feature 770, most clearly shown in Figure 22B and the linking portion and a second making joiner feature 780 on the handle body 218.

A further feature of this embodiment is that the linking portion 710 is in two pieces providing a telescopic action.

### Operation of the Device

Use or operation of the delivery device assembly 10 will now be described. The operation will be described with reference to the embodiment of Figures 1 to 18 but is the same for the embodiment of Figures 19 to 24.

Referring first to Figure 1A and its companion Figure 1B, the delivery device 11 is shown together with a sheath assembly 500 in a configuration ready for use. The combination of the delivery device 11 and the sheath assembly, or introducer, is the delivery device assembly 10.

Typically, one of the first major steps in a procedure undertaken by a vascular surgeon would be to introduce a guide wire into a blood vessel, such as the femoral artery, using the Seldinger technique. This technique involves creating a surgical opening in the vessel of the needle and inserting a wire guide into the vessel through a bore of the needle. The needle is then withdrawn leaving the guide wire in place. The delivery device assembly 10, as shown in Figures 1A and 1B, is then inserted over the guide wire and into the vessel.

Once the surgeon has positioned the proximal end 12 of the delivery device assembly 10 near the target delivery area for the endograft 5, the sheath assembly 500 can be withdrawn to the position shown in Figures 2A and 2B. In this position, the sheath 510 of the sheath assembly 500 has been pulled back over the compressed endograft for stent graft 5 so as to expose it, as is shown in Figure 2B. This step is conducted by "grounding" the handle body 218 while pulling the valve body 540 of the sheath assembly 500 in a distal direction (away from the patient). Importantly, this step also engages the linking portion 710 with the seal housing assembly 540 as there is a transition from the position shown in Figure 9 to the position shown in Figures 10A and 10B.

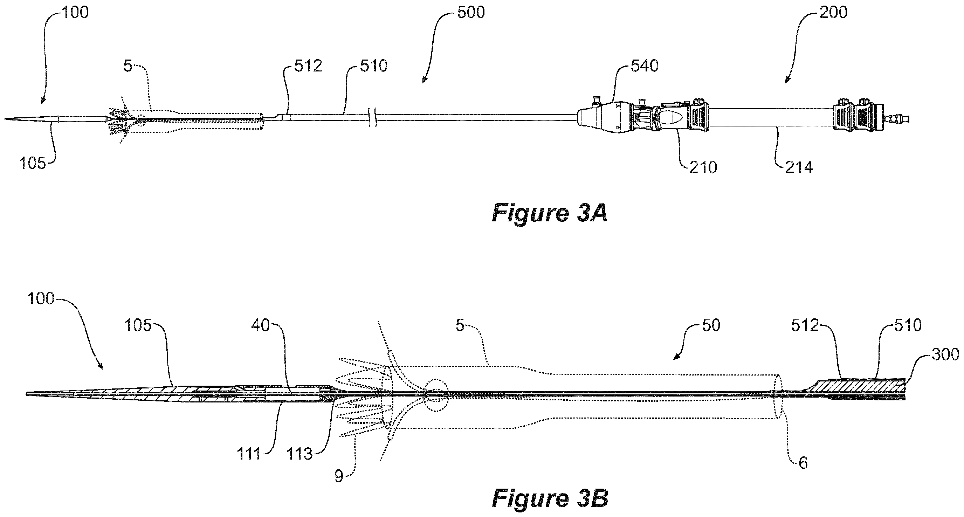

Typically, a next step in operating the delivery device 10 would be causing the stent graft to expand from its reduced condition to an expanded condition. This next step causes removal of a reducing trigger wire. This moves an end of the reducing trigger wire free from the endograft 5 allowing it to expand. The expanded condition can be seen in Figures 3A and 3B.

Other steps to deploy the endograft 5 into the patient are generally similar to the steps described in the applicant's earlier WO-1998/053761 entitled "A Prosthesis and a Method and Means of Deploying a Prosthesis".

After this step, the tip assembly 100 is retracted towards the sheath assembly 500 such that the sheath 510 ends up in a position against or over the tip retriever 113, as shown in Figure 5B, so that the entire delivery device 10 can safely be withdrawn through and out from within the deployed stent graft 5. This retraction step is illustrated in the transition from Figure 10A to Figure 11. Retraction is limited by the extent to which the linking portion 710 can slide with respect to the handle body 218. More specifically, the abutment between the flange 217 on the handle body and the tabs 712 on the linking portion 710, most clearly shown in Figures 15A and 15B, limits the extent of retraction. The limitation of the retraction is such that disengagement between the pusher catheter 300 and the pusher catheter seal 550 is prevented. This prevents severe blood loss occurring.

Operation of other embodiments of the invention, including the embodiment of Figures 19 to 24 would be largely similar.

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the invention is not restricted in its use to the particular application described. Neither is the present invention restricted in its preferred embodiment with regard to the particular elements and/or features described or depicted herein. It will be appreciated that the invention is not limited to the embodiment or embodiments disclosed, but is capable of numerous rearrangements, modifications and substitutions without departing from the scope of the invention as set forth and defined by the following claims.

The disclosures in Australian patent application number 2018/900064 and United States patent application number 62/615,720, from which this application claims priority, and in the abstract accompanying this application are incorporated herein by reference.

## Claims

1. An endoluminal delivery device assembly comprising:
an introducer including a seal housing assembly and an elongate sheath extending proximally from the seal housing assembly, the seal housing assembly housing a pusher catheter seal;
a delivery device including a handle body and an elongate pusher catheter extending proximally from the handle body, the pusher catheter having a pusher catheter external surface, the pusher catheter extending through the introducer such that the pusher catheter external surface slidably engages with the pusher catheter seal;
a tip assembly at a proximal end of the delivery device;
a guide wire catheter extending through the handle body and through the pusher, the guide wire catheter being affixed at a proximal end thereof to the tip assembly;
a medical device receiving portion for receiving the medical device between the tip assembly and the pusher; and
a linking portion for linking the handle body and the seal housing assembly together, the linking portion mounted between the handle body and the seal housing assembly, the linking portion having an unlinked condition and a linked condition,
whereby, in the linked condition, sliding movement between the pusher catheter and the pusher catheter seal is limited such that disengagement between the pusher catheter external surface and the pusher catheter seal is prevented.

2. An assembly as claimed in claim 1, wherein the linking portion is slidably mounted to the handle body.

3. An assembly as claimed in claim 1 or 2, wherein in the linked condition, the linking portion is joined to the seal housing assembly.

4. An assembly as claimed in claim 1, 2 or 3, comprising a joiner assembly for joining the linking portion to the seal housing assembly.

5. An assembly as claimed in claim 4, wherein the joiner assembly comprises:
a first mating joiner feature on the linking portion; and
a second mating joiner feature on seal housing assembly.

6. An assembly as claimed in any one of claims 1, 2 or 3, comprising a joiner assembly for joining the linking portion to the handle body.

7. An assembly as claimed in claim 6, wherein the joiner assembly comprises:
a first mating joiner feature on the linking portion; and a second mating joiner feature on handle body.

8. An assembly as claimed in any preceding claim, wherein the pusher catheter includes a proximal end, and wherein the assembly comprises:
an first condition in which the proximal end of the pusher catheter is spaced from the tip assembly at a first spacing for accommodating a compressed medical device; and
a second condition in which the proximal end of the pusher catheter is spaced from the tip assembly at a second spacing, the second spacing shorter than the first spacing.

9. An assembly as claimed in claim 8, wherein the sheath includes a proximal end, and wherein the assembly has a third condition in which the proximal end of the sheath is adjacent to the tip assembly.

10. An assembly as claimed in any preceding claim, wherein in the linked condition, the limited sliding movement between the pusher catheter and the pusher catheter seal is sufficient to bring the proximal end of the sheath into engagement with the tip assembly.

11. An assembly as claimed in any preceding claim, comprising a lock, the lock comprising :
a locking actuator having a locking projection; and
a locking recess,
wherein the lock has a locked condition in which the locking projection engages the locking recess thereby locking the linking portion to the handle body so as to prevent relative sliding movement.

12. An assembly as claimed in claim 11, wherein the actuator is moveable to an unlocked condition in which the locking projection is disengaged from the locking recess.

13. An assembly as claimed in any preceding claim, wherein in the unlinked condition, the linking portion is slidably mounted to the seal housing assembly.

14. An assembly as claimed in claim 13, wherein the linking portion is slidably mounted to the seal housing assembly.

15. An assembly as claimed in claim 13 or 14, wherein in the linked condition, the linking portion is joined to the handle body.
